# EUROPEAN PATENT APPLICATION

(11) **EP 3 441 058 A1**
(43) Date of publication of application: **13.02.2019**
(21) Application number: 18187753.1
(22) Date of filing: 07.08.2018
(51) Int. Cl.: A61K 6/083, A61K 6/00

(54) **GLASS INONOMER DENTAL CEMENT**

(30) Priority: 10.08.2017 JP 2017155688
(71) Applicant: GC Corporation, Sunto-gun, Shizuoka 410-1307 (JP)
(72) Inventor: TACHIBANA, Toshihiko, Tokyo 174-8585 (JP); KONO, Tomoki, Tokyo 174-8585 (JP); YOSHIMITSU, Ryosuke, Tokyo 174-8585 (JP); ARITA, Akishi, Tokyo 174-8585 (JP); NAKAYAMA, Mizuki, Tokyo 174-8585 (JP)
(74) Representative: Müller-Boré & Partner Patentanwälte PartG mbB

(57) **Abstract**

A dental cement includes: a first component containing a glass powder; a second component containing a polycarboxylic acid-based polymer, an organic polybasic acid, and water. The glass powder contains zinc and silicon. A solubility of a salt of a conjugate base of the organic polybasic acid and zinc ions in water at 20°C is greater than or equal to 1 g/100 mL.

## Description

The present invention relates to a dental cement.

A glass ionomer cement generally includes a powder component containing a fluoroaluminosilicate glass powder and a liquid component containing a polycarboxylic acid-based polymer and water. When the powder component and the liquid component are mixed, due to an acid-base reaction of the fluoroaluminosilicate glass powder with the polycarboxylic acid-based polymer, Al³⁺ eluted from the fluoroaluminosilicate glass powder and a conjugate base of the polycarboxylic acid-based polymer are ionically crosslinked, and the glass ionomer cement hardens.

Conventionally, tartaric acid is added to a liquid component (see, for example, Patent Documents 1 and 2). Adding tartaric acid to the liquid component brings a buffering action of pH and makes it easier to maintain low pH (acidic condition). Therefore, Al³⁺ is eluted from the fluoroaluminosilicate glass powder at an appropriate rate, and the glass ionomer cement hardens. As a result, the glass ionomer cement can be hardened in a time suitable for clinical use.

On the other hand, it is desired to enhance the effect of suppressing tooth demineralization of a dental cement.

### [Related-Art Documents]

### [Patent Documents]

[Patent Document 1] Japanese Unexamined Patent Application Publication No. S57-2210
[Patent Document 2] Japanese Unexamined Patent Application Publication No. S62-67008

Here, in order to enhance the effect of suppressing tooth demineralization of a glass ionomer cement, it is considered that a dental cement is prepared by adding a glass powder containing zinc instead of a fluoroaluminosilicate glass powder.

However, in such a case, when the powder component and the liquid component are mixed, due to an acid-base reaction of the glass powder with the polycarboxylic acid-based polymer, zinc ions eluted from the glass powder react with tartrate ions ((CH(OH)COO-)₂), and zinc tartrate is generated which is sparingly soluble in water. Thus, hardening of the dental cement is delayed, and there is a problem that the dental cement cannot be hardened in a time suitable for clinical use.

Here, the solubility of zinc tartrate in water at 20°C is 0.022 g/100 mL.

An object in one aspect of the present invention is to provide a dental cement having a large effect of suppressing tooth demineralization and that can be hardened in a time suitable for clinical use.

According to one aspect of the present invention, a dental cement includes: a first component containing a glass powder; and a second component containing a polycarboxylic acid-based polymer, an organic polybasic acid, and water, wherein the glass powder contains zinc and silicon, and wherein a solubility of a salt of a conjugate base of the organic polybasic acid and zinc ions in water at 20°C is greater than or equal to 1 g/100 mL.

According to one aspect of the present invention, it is possible to provide a dental cement having a large effect of suppressing tooth demineralization and that can be hardened in a time suitable for clinical use.

In the following, an embodiment for carrying out the present invention will be described.

### <Dental cement>

A dental cement according to the present embodiment includes a first component containing a glass powder and a second component containing a polycarboxylic acid-based polymer, an organic polybasic acid, and water.

The glass powder contains zinc and silicon. Therefore, when the first component and the second component are mixed, due to an acid-base reaction of the glass powder with the polycarboxylic acid-based polymer, Zn²⁺ eluted from the glass powder and a conjugate base of the polycarboxylic acid-based polymer are ionically crosslinked and hardened.

The solubility of a salt of a conjugate base of the organic polybasic acid and zinc ions in water at 20°C is greater than or equal to 1 g/100 mL, is preferably greater than or equal to 5 g/100 mL, and is more preferably greater than or equal to 10 g/100 mL. If the solubility of the salt of the conjugate base of the organic polybasic acid and zinc ions in water at 20°C is less than 1 g/100 mL, when the first component and the second component are mixed, the conjugate base of the organic polybasic acid reacts with zinc ions to generate a salt sparingly soluble in water is generated, and it is impossible to harden the dental cement in a time suitable for clinical use.

### <First Component>

The first component may be either a powder component or a liquid component.

The liquid component is preferably a paste dispersed in a dispersion medium such that the glass powder can be mixed with water.

### <Glass Powder>

The glass powder contains zinc and silicon, and, preferably, may further contain fluorine. This enhances the effect of preventing tooth decay of the dental cement.

The content of zinc in the glass powder is preferably in a range of from 10% to 60% by mass, and is more preferably in a range of from 20% to 50% by mass, in terms of zinc oxide (ZnO). When the content of zinc in the glass powder in terms of zinc oxide (ZnO) is greater than or equal to 10% by mass, the effect of suppressing tooth demineralization of the dental cement is enhanced. When the content of zinc in the glass powder in terms of zinc oxide (ZnO) is less than or equal to 60% by mass, the transparency of the glass powder is enhanced.

The content of silicon in the glass powder is preferably in a range of from 15% to 50% by mass, and is more preferably in a range of from 20% to 40% by mass, in terms of silicon oxide (SiO₂). Here, silicon serves to form a network in glass. When the content of silicon in the glass powder in terms of silicon oxide (SiO₂) is greater than or equal to 15% by mass, the transparency of the glass powder is enhanced. When the content of silicon in the glass powder in terms of silicon oxide (SiO₂) is less than or equal to 50% by mass, a hardening time of the dental cement becomes more appropriate.

The content of fluorine (F) in the glass powder is preferably in a range of from 1% to 30% by mass, and is more preferably in a range of from 3% to 20% by mass. When the content of fluorine (F) in the glass powder is greater than or equal to 1%, the effect of preventing tooth decay of the dental cement is enhanced. When the content of fluorine (F) in the glass powder is less than or equal to 30% by mass, a hardening time of the dental cement becomes more appropriate.

The glass powder may further contain aluminum, calcium, phosphorus, strontium, lanthanum, sodium, potassium or the like.

The content of calcium in the glass powder is preferably in a range of from 0% to 30% by mass, and is more preferably in a range of from 5% to 20% by mass, in terms of calcium oxide (CaO). When the glass powder contains calcium, the operability of the dental cement is improved.

The content of phosphorus in the glass powder is preferably in a range of from 0% to 10% by mass, and is more preferably in a range of from 0% to 5% by mass, in terms of phosphorus oxide (V) (P₂O₅). When the glass powder contains phosphorus, the operability of the dental cement is improved.

The content of strontium in the glass powder is preferably in a range of from 0% to 40% by mass, and is more preferably in a range of from 10% to 30% by mass, in terms of strontium oxide (SrO). When the glass powder contains strontium, the X-ray contrast property of a hardened substance of the dental cement is enhanced.

The content of lanthanum in the glass powder is preferably in a range of from 0% to 50% by mass, and is more preferably in a range of from 10% to 40% by mass in terms of lanthanum oxide (La₂O₃). When the glass powder contains lanthanum, the resistance to acids of a hardened substance of the dental cement is enhanced.

The content of sodium in the glass powder is preferably in a range of from 0% to 15% by mass, and is more preferably in a range of from 1% to 10% by mass, in terms of sodium oxide (Na₂O). When the glass powder contains sodium, the refractive index of the glass powder is lowered, and the transparency of the glass powder is enhanced.

The content of potassium in the glass powder is preferably in a range of from 0% to 10% by mass, and is more preferably in a range of from 1% to 5% by mass, in terms of potassium oxide (K₂O). When the glass powder contains potassium, the refractive index of the glass powder is lowered, and the transparency of the glass powder is enhanced.

### <Method for Producing Glass Powder>

The glass powder can be produced by, after melting a material composition containing a zinc compound and a silicon compound, pulverizing the material composition.

Examples of the zinc compound include, but are not limited to, zinc oxide, zinc fluoride, and the like, and two or more kinds may be used in combination as the zinc compound.

Examples of the silicon compound include, but are not limited to, anhydrous silicic acid and the like, and two or more kinds may be used in combination as the silicon compound.

The material composition may further contain a substance such as a fluorine compound.

Examples of the fluorine compound include, but are not limited to, calcium fluoride, strontium fluoride, sodium fluoride, and the like, and two or more kinds may be used in combination as the fluorine compound.

Note that each compound in the material composition may be mixed in accordance with a composition of the glass powder.

### <Second Component>

Although the second component is a liquid component, the liquid component may be in either a liquid state or a paste state.

### <Polycarboxylic Acid-Based Polymer>

Examples of the polycarboxylic acid-based polymer include, but are not limited to, a homopolymer or copolymer of an α,β-unsaturated carboxylic acid.

Examples of the α,β-unsaturated carboxylic acid constituting the polycarboxylic acid-based polymer include acrylic acid, methacrylic acid, 2-chloroacrylic acid, 3-chloroacrylic acid, aconitic acid, mesaconic acid, maleic acid, itaconic acid, fumaric acid, glutaconic acid, citraconic acid, and the like, and two or more kinds of these acids may be used in combination as the α,β-unsaturated carboxylic acid. Among these acids, acrylic acid or itaconic acid is particularly preferable.

The polycarboxylic acid-based polymer may be a copolymer of an α,β-unsaturated carboxylic acid and a component that is copolymerizable with the α,β-unsaturated carboxylic acid.

Examples of the component that is copolymerizable with an α,β-unsaturated carboxylic acid include acrylamide, acrylonitrile, a methacrylic ester, acrylates, vinyl chloride, allyl chloride, vinyl acetate, and the like, and two or more kinds may be used in combination.

In this case, the proportion of the α,β-unsaturated carboxylic acid to the monomer constituting the polycarboxylic acid-based polymer is preferably greater than or equal to 50% by mass.

The content of the polycarboxylic acid-based polymer in the second component is preferably in a range of from 5% to 60% by mass. When the content of the polycarboxylic acid-based polymer in the second component is greater than or equal to 5% by mass, a hardening time of the dental cement becomes more appropriate. When the content of the polycarboxylic acid-based polymer in the second component is less than or equal to 60% by mass, the operability of the dental cement is enhanced.

Note that at least part of the polycarboxylic acid-based polymer in the second component may be a powder.

### <Organic Polybasic Acid>

The organic polybasic acid is not particularly limited as long as the solubility of a salt of its conjugate base and zinc ions in water at 20°C is greater than or equal to 1 g/100 mL. Examples of the organic polybasic acid include a polybasic carboxylic acid such as citric acid, malic acid, succinic acid, or gluconic acid, and ascorbic acid, and the like, and two or more kinds of these acids may be used in combination as the organic polybasic acid.

The content of the organic polybasic acid in the second component is preferably in a range of from 5% to 30% by mass. When the content of the organic polybasic acid in the second component is greater than or equal to 5% by mass, a hardening time of the dental cement becomes more appropriate. When the content of the organic polybasic acid in the second component is less than or equal to 30% by mass, the strength of a hardened substance of the dental cement is enhanced.

The content of water in the second component is preferably in a range of from 30% to 70% by mass. When the content of water in the second component is greater than or equal to 30% by mass, a hardening time of the dental cement becomes more appropriate. When the content of water in the second component is less than or equal to 70% by mass, the strength of a hardened substance of the dental cement is enhanced.

Various agents such as an antibacterial agent, a fluorescent agent, a perfume, and a pigment may be added as needed to the dental cement according to the present embodiment.

### <Preparation of Kneaded Substance of Dental Cement>

The mass ratio of the first component to the second component when preparing a kneaded substance of the dental cement is preferably between 1 and 5. When the mass ratio of the first component to the second component is greater than or equal to 1, the strength of a hardened substance of the dental cement is enhanced. When the mass ratio of the first component to the second component is less than or equal to 5, the operability of the dental cement is enhanced.

### Examples

In the following, the present invention will be described in details with reference to Examples and Comparative Examples. Note that the present invention is not limited to Examples.

### <Preparation of Glass Powder>

After zinc oxide (ZnO), anhydrous silicic acid (SiO₂), calcium fluoride (CaF₂), lanthanum oxide (Za₂O₃), aluminum fluoride (AlF₃), strontium fluoride (SrF₂), sodium fluoride (NaF), aluminum phosphate (AlPO₄) and aluminum oxide (Al₂O₃) were mixed at a predetermined ratio, and the mixture was sufficiently mixed and stirred using a mortar to obtain a material composition. After the material composition was placed in a platinum crucible, it was placed in an electric furnace. The electric furnace was heated to 1300°C, and the material composition was melted and sufficiently homogenized. Subsequently, the material composition was poured into water to obtain aggregated glass. Using a ball mill made of alumina, the aggregated glass was pulverized for 20 hours and then it was caused to pass through a sieve of 120 meshes to obtain glass powders 1 to 4 as the first components.

### <Compositions of Glass Powders>

Using a fluorescent X-ray analyzer ZSX Primus II (manufactured by Rigaku Corporation), the glass powders 1 to 4 were analyzed to find their compositions.

Table 1 indicates the compositions of the glass powders 1 to 4 (unit: mass%).

**Table 1**

| | GLASS POWDER | | | |
|---|---|---|---|---|
| | 1 | 2 | 3 | 4 |
| Zn | 49.1 | 23.5 | 11.2 | |
| F | 3.6 | 4.4 | 4.2 | 5.0 |
| Al | | 5.1 | 9.9 | 18.1 |
| Si | 33.2 | 21.4 | 26.2 | 35.3 |
| Ca | 14.1 | 8.9 | 10.6 | 7.7 |
| P | | 5.3 | | 8.6 |
| Sr | | | 16.3 | 17.6 |
| La | | 31.4 | 20.1 | |
| Na | | | 1.5 | 77 |
| TOTAL | 100.0 | 100.0 | 100.0 | 100.0 |

Note that the contents of Zn, Si, Ca, La, Al, Sr, Na and P are respectively the contents in terms of ZnO, SiO₂, CaO, La₂O₃, Al₂O₃, SrO, Na₂O, and P₂O₅.

### <Preparation of Liquid>

The components indicated in Table 2 were mixed to obtain liquids 1 to 8 as the second components.

**Table 2**

| | LIQUID | | | | | | | |
|---|---|---|---|---|---|---|---|---|
| | 1 | 2 | 3 | 4 | 5 | 6 | 7 | 8 |
| POLYACRYLIC ACID | 40 | 40 | 40 | 40 | 40 | 40 | 40 | 40 |
| WATER | 50 | 40 | 50 | 40 | 50 | 40 | 50 | 40 |
| CITRIC ACID | 10 | 20 | | | | | | |
| MALIC ACID | | | 10 | 20 | | | | |
| GLUCO NIC ACID | | | | | 10 | 20 | | |
| TARTARIC ACID | | | | | | | 10 | 20 |
| TOTAL | 100.0 | 100.0 | 100.0 | 100.0 | 100.0 | 100.0 | 100.0 | 100.0 |

Here, the solubility of a salt of a conjugate base of each organic polybasic acid and zinc ions in water at 20°C is as follows.
Citric acid (zinc citrate): 10 g/100 mL
Malic acid (zinc malate): ≥ 1 g/100 mL
Acetic acid (zinc acetate): 30 g/100 mL
Tartaric acid (zinc tartrate): 0.022 g/100 mL

### [Examples 1-1 to 1-6 and Comparative Examples 1-1 and 1-2]

For each of Examples 1-1 to 1-6 and Comparative Examples 1-1 and 1-2 indicated in Table 3, the effect of suppressing tooth demineralization and the hardening time of the dental cement were evaluated.

### <Preparation of Kneaded Substance of Dental Cement>

The glass powder 1 and the liquids 1 to 8 were mixed such that the mass ratios of the glass powder 1 to the respective liquids 1 to 8 were 2, and then kneaded to obtain kneaded substances of the dental cements.

### <Effect of Suppressing Tooth Demineralization>

While water was poured, bovine dentine was polished by #1200 water-resistant abrasive paper. To the flat polished surface, a polytetrafluoroethylene seal, having a hole of which diameter is 3 mm, was attached. The kneaded substance of the dental cement was applied to half of the face of the hole, and it was left to stand in a thermostatic chamber at 37°C and 100% RH for 24 hours to harden the kneaded substance of the dental cement.

The bovine dentin, on which the hardened substance of the dental cement was formed, was immersed in a demineralized liquid (50 mM of acetic acid, 1.5 mM of calcium chloride, of 0.9 mM potassium dihydrogen phosphate, pH 4.5) at 37°C for 24 hours. The other half of the face of the hole, in contact with the demineralized liquid and on which the hardened substance of the dental cement was not formed, was tested as a test surface.

Using a precision cutting machine, the bovine dentin, on which the hardened substance of the dental cement was formed, was cut such that the thickness became 1 mm, and a test object was obtained.

Using an X-ray inspection apparatus, the test object was photographed by a transmission method. Using image processing software, the photographed image was analyzed to find the amount of mineral loss and to evaluate the effect of suppressing tooth demineralization.

The criteria for determining the effects of suppressing tooth demineralization are as follows. Note that as the value of the amount of mineral loss decreases, the effect of suppressing tooth demineralization increases.
Excellent: When the amount of mineral loss is less than 2000 volume% ·µm
Good: When the amount of mineral loss is greater than or equal to 2000 volume% ·um and less than 2500 volume% ·µm
Bad: When the amount of mineral loss is greater than or equal to 2500 volume% ·µm

Here, the effect of suppressing tooth demineralization was evaluated in a manner similar to that described above except that the kneaded substance of the dental cement was not applied at all. As a result, the amount of mineral loss was greater than or equal to 4302 vol% ·µm.

### <Hardening Time>

A mold (8 mm × 75 mm × 100 mm) adjusted to be at 23°C was placed on an aluminum foil, and the mold was filled with the kneaded substance of the dental cement up to the height of the upper surface of the mold. 60 seconds after the end of kneading, the kneaded substance of the dental cement was allowed to stand in a constant temperature layer at 37°C and 100% RH to harden the kneaded substance of the dental cement. 90 seconds after the end of kneading, 400 g of a Vicat needle was lowered vertically onto the surface of the hardened substance of the dental cement and it was maintained for 5 seconds. This operation was performed at intervals of 10 seconds to find the time until the dent by the Vicat needle became not a perfect circle (_see ISO 9917-1 Water-based cements Part1: Powder/liquid acid-base cements 8.1 Net setting time).

The criteria for determining the hardening times are as follows.
Excellent: When the hardening time is greater than or equal to 1 minute 30 seconds and less than or equal to 6 minutes
Good: When the hardening time is greater than 6 minutes seconds and less than or equal to 10 minutes
Bad: When the hardening time is less than 1 minute 30 seconds or greater than 10 minutes

Table 3 indicates the evaluation results of the effect of suppressing tooth demineralization and the hardening time of the dental cement for each of Examples 1-1 to 1-6 and Comparative Examples 1-1 and 1-2.

**Table 3**

| | Example | | | | | | Comparative Example | |
|---|---|---|---|---|---|---|---|---|
| | 1-1 | 1-2 | 1-3 | 1-4 | 1-5 | 1-6 | 1-1 | 1-2 |
| GLASS POWDER | 1 | 1 | 1 | 1 | 1 | 1 | 1 | 1 |
| LIQUID | 1 | 2 | 3 | 4 | 5 | 6 | 7 | 8 |
| HARDENING PROPERTY | EXCELLENT | EXCELLENT | EXCELLENT | EXCELLENT | EXCELLENT | EXCELLENT | BAD | BAD |
| HARDENING TIME [MINUTES' SECONDS] | 2'30 | 2'50 | 3'10 | 3'20 | 4'50 | 4'50 | 21'10 | 20'30 |
| EFFECT OF SUPPRESSING TOOTH DEMINERALIZATION | EXCELLENT | EXCELLENT | EXCELLENT | EXCELLENT | EXCELLENT | EXCELLENT | EXCELLENT | EXCELLENT |
| AMOUNT OF MINERAL LOSS [VOLUME%· *µ*m] | 1283 | 1165 | 1321 | 1228 | 1374 | 1298 | 1325 | 1446 |

As can been seen from Table 3, the dental cements of Examples 1-1 to 1-6 have a large effect of suppressing tooth demineralization and can be hardened in a time suitable for clinical use.

In contrast, the dental cements of Comparative Examples 1-1 and 1-2 cannot be hardened in a time suitable for clinical use because the liquids 7 and 8 contain tartaric acid.

### [Examples 2-1 to 2-6 and Comparative Examples 2-1 and 2-2]

For each of Examples 2-1 to 2-6 and Comparative Examples 2-1 and 2-2, the effect of suppressing tooth demineralization and the hardening time of the dental cement were evaluated in a manner similar to that in Examples 1-1 to 1-6 and Comparative Examples 1-1 and 1-2 except that the glass powder 2 was used instead of the glass powder 1.

Table 4 indicates the evaluation results of the effect of suppressing tooth demineralization and the hardening time of the dental cement for each of Examples 2-1 to 2-6 and Comparative Examples 2-1 and 2-2.

**Table 4**

| | Example | | | | | | Comparative Example | |
|---|---|---|---|---|---|---|---|---|
| | 2-1 | 2-2 | 2-3 | 2-4 | 2-5 | 2-6 | 2-1 | 2-2 |
| GLASS POWDER | 2 | 2 | 2 | 2 | 2 | 2 | 2 | 2 |
| LIQUID | 1 | 2 | 3 | 4 | 5 | 6 | 7 | 8 |
| HARDENING PROPERTY | EXCELLENT | EXCELLENT | EXCELLENT | EXCELLENT | EXCELLENT | EXCELLENT | BAD | BAD |
| HARDENING TIME [MINUTES' SECONDS] | 3'40 | 4'00 | 4'30 | 4'50 | 5'40 | 6'00 | 17'10 | 17'50 |
| EFFECT OF SUPPRESSING TOOTH DEMINERALIZATION | EXCELLENT | EXCELLENT | EXCELLENT | EXCELLENT | GOOD | EXCELLENT | EXCELLENT | GOOD |
| AMOUNT OF MINERAL LOSS [VOLUME%· *µ*m] | 1804 | 1783 | 1776 | 1878 | 2034 | 1986 | 1982 | 2018 |

As can been seen from Table 4, the dental cements of Examples 2-1 to 2-6 have a large effect of suppressing tooth demineralization and can be hardened in a time suitable for clinical use.

In contrast, the dental cements of Comparative Examples 2-1 and 2-2 cannot be hardened in a time suitable for clinical use because the liquids 7 and 8 contain tartaric acid.

### [Examples 3-1 to 3-6 and Comparative Examples 3-1 and 3-2]

For each of Examples 3-1 to 3-6 and Comparative Examples 3-1 and 3-2, the effect of suppressing tooth demineralization and the hardening time of the dental cement were evaluated in a manner similar to that in Examples 1-1 to 1-6 and Comparative Examples 1-1 and 1-2 except that the glass powder 3 was used instead of the glass powder 1.

Table 5 indicates the evaluation results of the effect of suppressing tooth demineralization and the hardening time of the dental cement for each of Examples 3-1 to 3-6 and Comparative Examples 3-1 and 3-2.

**Table 5**

| | Example | | | | | | Comparative Example | |
|---|---|---|---|---|---|---|---|---|
| | 3-1 | 3-2 | 3-3 | 3-4 | 3-5 | 3-6 | 3-1 | 3-2 |
| GLASS POWDER | 3 | 3 | 3 | 3 | 3 | 3 | 3 | 3 |
| LIQUID | 1 | 2 | 3 | 4 | 5 | 6 | 7 | 8 |
| HARDENING PROPERTY | EXCELLENT | EXCELLENT | EXCELLENT | EXCELLENT | EXCELLENT | GOOD | BAD | BAD |
| HARDENING TIME [MINUTES' SECONDS] | 4'10 | 4'30 | 4'50 | 4'40 | 5'50 | 6'50 | 15'20 | 15'10 |
| EFFECT OF SUPPRESSING TOOTH DEMINERALIZATION | GOOD | GOOD | GOOD | GOOD | GOOD | GOOD | GOOD | GOOD |
| AMOUNT OF MINERAL LOSS [VOLUME%· *µ*m] | 2224 | 2187 | 2314 | 2287 | 2399 | 2448 | 2351 | 2468 |

As can been seen from Table 5, the dental cements of Examples 3-1 to 3-6 have a large effect of suppressing tooth demineralization and can be hardened in a time suitable for clinical use.

In contrast, the dental cements of Comparative Examples 3-1 and 3-2 cannot be hardened in a time suitable for clinical use because the liquids 7 and 8 contain tartaric acid.

### [Comparative Examples 4-1 to 4-8]

For each of Comparative Examples 4-1 and 4-8, the effect of suppressing tooth demineralization and the hardening time of the dental cement were evaluated in a manner similar to that in Comparative Examples 1-1 and 1-2 except that the glass powder 4 was used instead of the glass powder 1.

Table 6 indicates the evaluation results of the effect of suppressing tooth demineralization and the hardening time of the dental cement for each of Comparative Examples 4-1 and 4-8.

**Table 6**

| | Comparative Example | | | | | | | |
|---|---|---|---|---|---|---|---|---|
| | 4-1 | 4-2 | 4-3 | 4-4 | 4-5 | 4-6 | 4-7 | 4-8 |
| GLASS POWDER | 4 | 4 | 4 | 4 | 4 | 4 | 4 | 4 |
| LIQUID | 1 | 2 | 3 | 4 | 5 | 6 | 7 | 8 |
| HARDENING PROPERTY | EXCELLENT | EXCELLENT | EXCELLENT | EXCELLENT | EXCELLENT | EXCELLENT | EXCELLENT | EXCELLENT |
| HARDENING TIME [MINUTES' SECONDS] | 5'30 | 6'00 | 4'00 | 4'30 | 4'30 | 4'50 | 4'20 | 4'00 |
| EFFECT OF SUPPRESSING TOOTH DEMINERALIZATION | BAD | BAD | BAD | BAD | BAD | BAD | BAD | BAD |
| AMOUNT OF MINERAL LOSS [VOLUME%· *µ*m] | 2978 | 2884 | 2994 | 3011 | 2961 | 3067 | 3103 | 3225 |

As can been seen from table 6, the dental cements of Comparative Examples 4-1 to 4-8 have a small effect of suppressing tooth demineralization because the glass powder 4 does not include zinc.

## Claims

1. A dental cement comprising:
a first component containing a glass powder; and
a second component containing a polycarboxylic acid-based polymer, an organic polybasic acid, and water,
wherein the glass powder contains zinc and silicon, and
wherein a solubility of a salt of a conjugate base of the organic polybasic acid and zinc ions in water at 20°C is greater than or equal to 1 g/100 mL.

2. The dental cement according to claim 1, wherein the organic polybasic acid is one or more acids selected from a group consisting of citric acid, malic acid, succinic acid, gluconic acid, and ascorbic acid.

3. The dental cement according to claim 1 or 2, wherein the glass powder further contains fluorine.
